Europäisches Patentamt

European Patent Office

Office européen des brevets

(19)

(11) Publication number: **0 241 208**

**A1**

(12) **EUROPEAN PATENT APPLICATION**

(21) Application number: **87302835.1**

(22) Date of filing: **01.04.87**

(51) Int. Cl.³: **C 12 N 15/00**
C 12 N 9/68, C 07 H 21/04
C 12 N 5/00, A 61 K 37/54

(30) Priority: **02.04.86 GB 8608016**
**02.04.86 GB 8608017**
**11.09.86 GB 8621868**
**21.02.87 GB 8704077**

(43) Date of publication of application:
**14.10.87 Bulletin 87/42**

(84) Designated Contracting States:
**BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Browne, Michael Joseph Beecham**
**Pharmaceuticals**
**Great Burgh Yew Tree Bottom Road**
**Epsom Surrey KT18 5XQ(GB)**

(72) Inventor: **Robinson, Jeffrey Hugh Beecham**
**Pharmaceuticals**
**Great Burgh Yew Tree Bottom Road**
**Epsom Surrey KT18 5XQ(GB)**

(74) Representative: **Valentine, Jill Barbara et al,**
**Beecham Pharmaceuticals Patents & Trade Marks Dept.**
**Great Burgh Yew Tree Bottom Road**
**Epsom Surrey KT18 5XQ(GB)**

(54) **Modified fibrinolytic enzyme.**

(57) A fibrinolytically active tissue-type plasminogen activator which has been modified in the region of the first kringle domain and optionally also the growth factor domain.

Apparent $M_r$ $(\times 10^{-3})$

90 —
65 —
56 —
38 —
30 —

Fig. 1

EP 0 241 208 A1

0241208

- 1 -

## Novel Compounds

The present invention relates to a modified
fibrinolytic enzyme, in particular modified tissue-type
plasminogen activator, its preparation, pharmaceutical
compositions containing it and its use in the treatment
of thrombotic disease.

The sequence of amino acids making up the enzyme
tissue-type plasminogen activator (t-PA) and the
nucleotide sequence for the cDNA which codes for t-PA
are known (see Pennica et al, 1983; Nature, 301, 214).
t-PA is known to have fibrinolytic activity.

Native t-PA is composed of a B or light (L) and an A or
heavy (H) chain.  The B chain contains the active site
of the enzyme.

It has been shown (Ny, T. et al, 1984; Proc. Natl.
Acad. Sci. U.S.A., 81, 5355) that the A chain exhibits
a number of structural and functional domains which are
homologous to structures found in other plasma
proteins:  two triple disulphide-bonded structures or
kringles, a growth-factor-like domain and a
fibronectin-finger-like domain.  The region from amino
acid residues 44 to 91 has been termed the ''growth
factor domain'' (Banyai, L. et al, FEBS Lett. 163
37,1983).  The genetic information which codes for the
major part of this domain, residues 51 to 86 inclusive,
and partially codes for residues 50 and 87, lies on a
single exon.

The region from the first to the last cysteine residue
of the first kringle structure, residues 92 to 173 inclusive, will be referred to herein as the first kringle domain.

The applicants have now identified modified forms of the t-PA enzyme which retain fibrinolytic activity.

According to the present invention there is provided a fibrinolytically active tissue-type plasminogen activator which has been modified in the region of the first kringle domain and optionally also the growth factor domain.

Suitable modifications may include removal or deletion of certain amino acid residues, or replacement of one or more amino acid residues with different amino acid residues.

The modified region may optionally extend in either or both directions beyond the first kringle domain as herein defined, up to and including residues 85 and 179 respectively.

In a first preferred aspect, the modification comprises the deletion of all or part of the first kringle domain.

In another aspect, the t-PA is modified in the region from amino acid residues 88 to 179 inclusive.

In a preferred aspect, the modification comprises the deletion of amino acid residues 92 to 179 inclusive.

Where the tissue-type plasminogen activator has also been modified in the region of the growth factor domain, the modified region may optionally extend beyond the first kringle domain as herein defined, up to and including residue 179. In a second preferred aspect, the modification preferably comprises the deletion of all or part of the first kringle and growth factor domains.

In another aspect, the t-PA is modified in the region from amino acid residues 51 to 179 inclusive.

In a preferred aspect, the modification comprises the deletion of amino acid residues 51 to 87 and 92 to 173 inclusive.

Alternatively, the modification may comprise the deletion of amino acid residues 51 to 173 inclusive.

As used herein, the term tissue-type plasminogen activator denotes a plasminogen activator of the group having the immunological properties defined for t-PA at the XXVIII Meeting of the International Committee on Thrombosis and Haemostasis, Bergamo, Italy, 27 July 1982.

The amino acid sequence of various forms of t-PA are known. The abovementioned Nature 1983 reference discloses the sequence for the L-chain and the mature S-chain forms of t-PA, also discussed by Vehar et.al., Biotechnology, 1984, 2, 1051-7 in which the processing of initially formed t-PA by removal of a pro-sequence to give the S-chain form is reported. Pohl et.al., FEBS letters, 1984, Vol. 168 No.1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue

- 4 -

at position -3 and U-chain t-PA has an N-terminal valine at position 4. It is understood that the tissue-type plasminogen activator modified in accordance with the present invention encompasses all such variant forms.

The modified t-PA of the invention may be derivatised to provide pharmaceutically useful conjugates analogous to known t-PA-containing conjugates, for example:

(a)  an enzyme-protein conjugate as disclosed in EP-A-0 155 388, in which the catalytic site on the enzyme which is responsible for fibrinolytic activity is blocked by a human protein attached thereto by way of a reversible linking group;

(b)  an enzyme-protein conjugate as disclosed in EP-A-0 152 736, comprising at least one optionally blocked fibrinolytic enzyme linked by way of a site other than the catalytic site responsible for fibrinolytic activity to at least one human protein;

(c)  a protein-polymer conjugate as disclosed in EP-A-0 183 503 comprising a pharmaceutically useful protein linked to at least one water soluble polymer by means of a reversible linking group; or

(d)  an enzyme conjugate as disclosed in EP-A-0 184 363 comprising a plurality of fibrinolytic enzymes linked together through the active centres thereof by means of a removable blocking group.

The modified t-PA of the invention may take the place of t-PA as the enzyme or (human) protein component, as appropriate, of any of the conjugates described above.

The modified t-PA of the invention or conjugate thereof can be further derivatised such that any catalytic site essential for fibrinolytic activity is optionally blocked by a removable blocking group.

The above mentioned derivatives of the modified t-PA may be used in any of the methods and compositions described hereinafter for the modified t-PA itself.

As used herein the expression 'removable blocking group' includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing modified tissue-type plasminogen activator according to the invention which process comprises expressing DNA encoding said modified tissue-type plasminogen activator in a recombinant host cell and recovering the modified tissue-type plasminogen activator product.

The modification of the t-PA can therefore be carried out by conventional genetic engineering techniques in which the cDNA which codes for t-PA is modified and the modified cDNA expressed in a prokaryote or eukaryote host.

The DNA polymer comprising a nucleotide sequence that encodes the modified t-PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

    i)    preparing a replicable expression vector capable, in a host cell, of expressing a DNA polymer comprising a nucleotide sequence that encodes said modified tissue-type plasminogen activator;

    ii)  transforming a host cell with said vector;

    iii) culturing said transformed host cell under conditions permitting expression of said DNA polymer to produce said modified tissue-type plasminogen activator; and

    iv)  recovering said modified tissue-type plasminogen activator.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate.  Thus, the DNA polymer may be prepared by the enzymatic ligation of chemically synthesised DNA fragments, by conventional methods such as those described by D. M. Roberts et al in Biochemistry 1985, 24, 5090-5098.  The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50µl or less with 0.1-10µg DNA.

The chemical synthesis may be performed generally as described hereinafter for the synthesis of an oligodeoxyribonucleotide.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50µl or less.

Enzymatic ligation of DNA fragments may be carried out using a DNA ligase such as T4 DNA ligase in an appropriate buffer at a temperature of 4°C to ambient, generally in a volume of 50µl or less.

DNA polymer which encodes the modified t-PA may be prepared by site-directed mutagenesis of the cDNA which codes for tissue-type plasminogen activator, by conventional methods such as those described by G. Winter et al in Nature 1982, 299, 756-758 or by Zoller and Smith 1982; Nucl. Acids Res., 10, 6487-6500, or deletion mutagenesis such as described by Chan and Smith in Nucl. Acids Res., 1984, 12, 2407-2419 or by G. Winter et al in Biochem. Soc. Trans., 1984, 12, 224-225. Expression of the modified cDNA can be carried out by conventional methods as described hereinafter.

The above described mutagenesis processes involve the use of oligodeoxyribonucleotides which can be designed according to the changes in the cDNA sequence required.

The oligodeoxyribonucleotides which may be used in such processes also form part of the invention.

The invention also provides a process for preparing an oligodeoxyribonucleotide of the invention, which process comprises the deprotection of a second oligodeoxyribonucleotide having the same sequence of bases as said oligodeoxyribonucleotide and in which all free hydroxy and amino groups are protected.

The oligodeoxyribonucleotides used in the process can be prepared by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

Solid supports which may be employed include conventional supports known in the art, for example kieselguhr-polydimethylacrylamide, silica, controlled-pore glass, or cellulose paper discs. The base at the 3'-end of the oligodeoxyribonucleotide to be prepared is attached, via a spacer group connected to the 3'-oxygen of the terminal 2'-deoxyribose unit, to the solid support and assembly of the oligodeoxyribonucleotide (in a protected form) is carried out in the 3'-> 5'-direction by cycles of synthesis, the required operations being performed either manually or by an automated instrument.

At the end of the synthesis, the protected oligodeoxyribonucleotide may be cleaved from the solid support either before, after, or at the same time as other deprotection steps that are carried out once the desired sequence of bases has been assembled, as described hereinbelow. Conveniently the oligodeoxyribonucleotide is removed from the solid support by treatment with a basic reagent such as aqueous ammonia at ambient or slightly elevated temperature, or with 1,1,3,3 -tetramethyl-guanidinium-syn- 2-nitrobenzaldoximate or similar reagents in aqueous dioxan at ambient or slightly elevated temperature.

Alternatively, the preparation may be carried out conventionally by phosphotriester chemistry in solution as described, for example, by C.B. Reese, Tetrahedron, 1978, 34, 3143-3179.

The required sequence of nucleotide bases in the oligodeoxyribonucleotide may be built up conventionally, for example as described in the aforementioned publications, by the condensation of appropriate mono-, di- or oligomeric nucleotide units in the presence of a suitable condensing agent such as 1-(mesitylene-sulphonyl)-3- nitro-1,2,4-triazole in a solvent such as pyridine at ambient or slightly elevated temperature (when phosphotriester chemistry is employed) or by a condensing agent such as tetrazole in acetonitrile at ambient temperature (when phosphite or phosphoramidite chemistry is used). Optionally a 'capping' step is introduced after each condensation step to inactivate any unreacted starting material containing a free 5'-hydroxy group. Such a capping step may suitably be carried out by an acylating agent such as acetic anhydride in 2,6-lutidine and 4-$\underline{N}$,$\underline{N}$-dimethylamino-pyridine in tetrahydrofuran at ambient temperature.

When phosphite or phosphoramidite chemistry is employed, the phosphorus (III) atom in the phosphite-triester internucleotide linkage created in each condensation step is oxidised, giving the corresponding phosphotriester linkage, before a further condensation step is carried out. Such oxidations may be carried out using a convenient oxidising agent, for example iodine in aqueous tetrahydrofuran in the presence of a base such as lutidine. The oxidation step is conveniently carried out after the optional 'capping' step as hereinbefore described.

- 11 -

During the synthesis of the oligodeoxyribonucleotide, the hydroxy groups in each of the internucleotide phosphodiester bridges may be protected by an aryl group or alkyl group conventionally employed for the purpose, for example 2- or 4-chlorophenyl, methyl, or 2-cyanoethyl, to prevent branching of the molecule. The aryl protecting groups are removed at the end of the synthesis by treatment with an agent conventionally employed for the purpose, for example 1,1,3,3-tetramethyl-guanidinium- _syn_ -2-nitrobenzaldoximate in an aqueous solvent such as aqueous dioxan at ambient temperature. The methyl groups may be removed at the end of the synthesis by treatment with triethylammonium thiophenolate in dioxan at ambient temperature. The 2-cyanoethyl groups may be removed at the end of the synthesis with a basic reagent such as triethylamine or tert-butylamine in pyridine at ambient temperature, or alternatively by treatment with concentrated aqueous ammonia at about 50°C (a step which simultaneously and advantageously removes the protecting groups present on the A, C and G bases as described hereinbelow).

The amino groups present in the A, C and G bases are protected by base-labile protecting groups, such as benzoyl for A and C, and isobutyryl for G. Such groups may be removed by treatment with basic reagents, for example ammonia at ambient or slightly elevated temperature, for example 50°C. The 5'-hydroxy group of the terminal ribose moiety is protected by an acid-labile group such as trityl, 4,4'-dimethoxytrityl or pixyl (9-phenyl-9-xanthyl), which is removed before each condensation step and at the end of synthesis. Such groups may be removed by treatment with an acidic reagent such as di- or trichloroacetic acid in an anhydrous solvent such as dichloromethane or chloroform, at ambient temperature.

Where the modification of t-PA comprises the deletion of the growth factor and first kringle domains, the DNA polymer is preferably prepared by ligating a first DNA molecule comprising a DNA sequence encoding the finger domain amino acid sequence with a second DNA molecule comprising a DNA sequence encoding the second kringle domain.

The DNA molecules may be obtained by digestion with suitable restriction enzymes of vectors carrying the required coding sequences.

In the preferred embodiment, a DNA molecule comprising the sequence encoding the finger domain may be obtained by the digestion with the restriction enzymes HindIII and MaeII of the plasmid pTRE24 disclosed in EP-A-0207589. This plasmid comprises the DNA sequence encoding t-PA but with the portion encoding amino acid residues 51 to 87 deleted. The digestion provides a DNA fragment (I) including portions encoding residues 1 to 50 and 88 to 90, and partially encoding residue 91:

```
                       MaeII site

                          |
    5' AGT ACC AGG GCC A    3'coding strand       (I)
    3' TCA TGG TCC CGG TGC 5'

                             |
       ser thr arg ala thr
       50  88  89  90  91
```

This fragment (I) may be blunt-ended and one or more linkers added, such as the BamHI linker from Amersham (DC1203). The linker is digested to provide a sticky end such as:

```
    5' CCC G        3'
    3' GGG CCT AG 5'
```

- 13 -

The second molecule may be obtained by digestion of pTRE15 disclosed in EP-A-0201153 with the enzymes MaeIII and SstI (SstI is a unique site in pTRE15). The digestion provides a fragment (II) encoding the second kringle and partly encoding the t-PA B-chain.

(II)

```
        MaeIII site                       SstI site
            |                                 |
    5' GT GAC TGC TAC ..........TGT GAG CT        3'
          ACG ATG ..........ACA C                 5'
            |                       |
          cys tyr                 cys
          180 181                 409
```

The two restriction fragments (molecules) may be joined by an oligonucleotide linker.

The preferred oligonucleotide linker is of structure (III):

```
    5' GA TCC GAG GGA AAC A        3'
    3'       G CTC CCT TTG TCA CTG 5'
```

The oligonucleotide linker (III) is novel and as such forms part of the invention.

The overhanging sequence at the 5′ end may be used to join the linker (III) to the fragment (I) by reconstructing the BamHI site, and the overhanging sequence at the 3′ end may be used to join the linker to the fragment (II) by reconstructing the MaeIII site.

- 14 -

The remainder of the t-PA B-chain and the vector functions may be provided by a fragment (IV) obtained by digestion of pTRE15 with HindIII and SstI.

The ligation of fragments (I), (II), (III) and (IV) may be carried out in a single step.

The resulting sequences at the ligation sites of fragments (I), (II) and (III) are believed to be as follows:

```
        (I) Join       (III)      Join (II)
5'... ACG CCC GGA TCC GAG GGA AAC AGT GAC TGC ... 3'
3'... TGC GGG CCT AGG CTC CCT TTG TCA CTG ACG ... 5'

           |                              |
          460                            727
```

Ligation of the SstI sticky end of the resulting molecule with that of fragment (IV) reforms the B-chain coding region.

The joining of fragments (I) and (II) using the linker (III) creates a DNA molecule which when expressed as protein is believed to have a similar sequence to that described in Pennica et al., except for the following alteration:

```
position 49  50  88  89  90  91  *   *   174 175
         lys ser thr arg ala thr pro gly ser glu
```

The expression of the DNA polymer encoding the modified t-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the modified t-PA, under ligating conditions.

The ligation of the linear segment and more than one DNA molecule may be carried out simultaneously or sequentially as desired.

Thus, the DNA polymer may be preformed or formed during the construction of the vector, as desired.

In particular, the replicable expression vector may be prepared by cleaving a vector compatible with the host cell to provide a linear DNA segment having an intact replicon, and combining said linear segment with the DNA polymer encoding the modified t-PA, intact or alternatively provided in two or more fragments, under ligating conditions.

The choice of vector will be determined in part by the host cell, which may be prokaryotic or eukaryotic. Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis et al cited above. Polymerisation and ligation may be performed as described above for the preparation of the DNA polymer. Digestion with restriction enzymes may be performed in an appropriate buffer at a temperature of $20^{\circ}-70^{\circ}C$, generally in a volume of 50µl or less with 0.1-10µg DNA.

The recombinant host cell is prepared, in accordance witn the invention, by transforming a host cell with a replicable expression vector of the invention under transforming conditions. Suitable transforming conditions are conventional and are described in, for example, Maniatis et al cited above, or ''DNA Cloning'' Vol. II, D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells.

The invention also extends to a host cell transformed with a replicable expression vector of the invention.

Culturing the transformed host cell under conditions permitting expression of the DNA polymer is carried out conventionally, as described in, for example, Maniatis et al and ''DNA Cloning'' cited above. Thus, preferably the cell is supplied with nutrient and cultured at a temperature below $45^{\circ}C$.

The modified t-PA expression product is recovered by conventional methods according to the host cell. Thus, where the host cell is bacterial, such as E. coli it may be lysed physically, chemically or enzymatically

- 17 -

and the protein product isolated from the resulting lysate. Where the host cell is mammalian, the product may generally be isolated from the nutrient medium.

The DNA polymer may be assembled into vectors designed for isolation of stable transformed mammalian cell lines expressing the modified t-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al.,European Patent Application No. 0093619, 1983).

It will be appreciated that, depending upon the host cell, the modified t-PA prepared in accordance with the invention may be glycosylated to varying degrees. Furthermore, as observed by Pohl et.al., Biochemistry, 1984, 23, 3701-3707, varying degress of glycosylation may also be found in unmodified, naturally occurring t-PA. The modified t-PA of the invention is understood to include such glycosylated variations.

In a preferred embodiment, a tissue-type plasminogen activator enzyme modified in the region of the first kringle domain is prepared by a process which comprises effecting a deletion mutagenesis upon the cDNA which codes for tissue-type plasminogen activator using an oligodeoxyribonucleotide comprising a nucleotide sequence which is complementary or corresponds to a region of the t-PA cDNA extending 5' from nucleotide 462 inclusive, said sequence being joined directly to a nucleotide sequence which is complementary or corresponds to a region extending 3' from nucleotide 727 inclusive of t-PA cDNA and expressing the modified cDNA in a prokaryote or eurokaryote host.

The oligodeoxyribonucleotide employed in the preferred embodiment is suitably at least 16 nucleotides in length, preferably at least 26 nucleotides in length. Suitably it contains at least 8 and preferably at least 12 nucleotides which are complementary to nucleotides on each side of the deletion region.

A preferred oligodeoxyribonucleotide is of sequence (V).

        5'd(TTCCCAAAGTAGCACGTGGCCCTGGT)3'          (V)

The oligodeoxyribonucleotide of sequence (V) is novel and as such forms part of the invention.

The 14 bases at the 5' end of the oligodeoxyribonucleotide (V) are complementary to t-PA cDNA nucleotides 740-727 inclusive and the 12 bases at the 3' end of the oligodeoxyribonucleotide (V) are complementary to the t-PA cDNA nucleotides 462-451 inclusive.

The oligonucleotide of sequence (V) can be used to delete the portion of cDNA which codes for amino acids 92 to 179 inclusive of t-PA. It is believed that the cDNA obtained following mutagenesis is similar to that of Pennica et al, 1983, Nature, 301, 214 with the following new nucleotide sequence:-

        5' - ACC AGG GCC ACG TGC TAC TTT GGG AA 3'
              |                 | |             |
        position 451          462 727          740

The t-PA obtained by expression of this cDNA is expected to have a similar amino acid sequence to that described in Pennica *et al.*, except for the following alteration:

```
    position 88          91 180             184
       |                 |  |                 |
          -thr-arg-ala-thr-cys-tyr-phe-gly-asn-
```

In another embodiment, a tissue-type plasminogen activator enzyme modified in the region of both the growth factor and the first kringle domains is prepared by a process which comprises effecting a deletion mutagenesis upon the cDNA which codes for tissue-type plasminogen activator using an oligodeoxyribonucleotide comprising a nucleotide sequence which is complementary or corresponds to a region of the t-PA cDNA extending 5′ from nucleotide 339 inclusive, said sequence being joined directly to a nucleotide sequence which is complementary or corresponds to a region extending 3′ from nucleotide 709 inclusive of t-PA cDNA and expressing the modified cDNA in a prokaryote or eurokaryote host.

The oligodeoxyribonucleotide employed in this embodiment is suitably at least 16 nucleotides in length, preferably at least 26 nucleotides in length. Suitably it contains at least 8 and preferably at least 12 nucleotides which are complementary to nucleotides on each side of the deletion region.

A preferred oligodeoxyribonucleotide is of sequence (VI).

     5'd(TGTTTCCCTCAGAACTTTTGACAGGC)3'      (VI)

The oligodeoxyribonucleotide of sequence (VI) is novel and as such forms part of the invention.

The 13 bases at the 5' end of the oligodeoxyribonucleotide (VI) are complementary to t-PA cDNA
nucleotides 721-709 inclusive and the 13 bases at the
3' end of the oligodeoxyribonucleotide (VI) are
complementary to the t-PA cDNA nucleotides 339-327
inclusive.

The oligodeoxyribonucleotide of sequence (VI) can be
used to delete the portion of cDNA which codes for
amino acids 51 to 173 inclusive of t-PA.  It is
believed that the cDNA obtained following mutagenesis
is similar to that of Pennica et al, 1983, Nature, 301,
214 with the following new nucleotide sequence:-

```
          5' - G CCT GTC AAA AGT TCT GAG GGA AAC A 3'
                |                 |  |           |
          position 327         339 709         721
```

The t-PA obtained by expression of this cDNA is
expected to have a similar amino acid sequence to that
described in Pennica et al., except for the following
alteration:

```
position 46                    50   174          178
                                |    |             |
                -val-pro-val-lys-ser-ser-glu-gly-asn-ser-
```

The modified t-PA of the invention comprises the
B-chain of native t-PA linked to t-PA A-chain modified
in the region of the first kringle domain and
optionally also the growth factor.  This modified t-PA
A-chain may be employed as one chain of a
fibrinolytically active hybrid protein such as
disclosed in EP-0155387.  The modified t-PA A-chain,
DNA encoding the modified t-PA A-chain and a hybrid
protein comprising the modified t-PA A chain linked to

024120

the B-chain of a fibrinolytically active protease, the catalytic site of which is optionally blocked by a removable blocking group, all form part of the invention. The modified t-PA A-chain may be prepared by separation from the B-chain thereof by mild reduction. Alternatively the modified A-chain may be prepared by expressing DNA encoding therefor in a recombinant host cell and recovering the modified t-PA A-chain product. The hybrid protein comprising the modified t-PA A-chain linked to the B-chain of a fibrinolytically active protease may be prepared by (a) mixing said A- and B-chains under oxidative conditions; or (b) ligating DNA encoding said A-chain to DNA encoding said B-chain and expressing the ligated DNA in a prokaryote or eukaryote host; and thereafter optionally blocking the catalytic site of the hybrid protein with a removable blocking group. The oxidation and reduction conditions are as generally described in EP-0155387. The hybrid protein may be used in any of the methods and compositions described hereinafter for the modified t-PA itself.

The modified t-PA of the invention is suitably administered in the form of a pharmaceutical composition.

Accordingly the present invention also provides a pharmaceutical composition comprising modified t-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified t-PA in solution and a local anaesthetic such as lignocaine to ease pain at the site of injection. Generally, the modified t-PA will be supplied in unit dosage form for example as a dry powder or water free concentrate in a hermetically sealed container such as an ampoule or sachette indicating the quantity of protein in activity units. Where the modified t-PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration.

The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a mature, fresh or nascent thrombus will generally receive a daily dose of from 0.01 to 10 mg/kg of body weight either by injection in for example up to five doses or by infusion.

- 23 -

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified t-PA of the invention.

The invention also provides modified t-PA of the invention for use as an active therapeutic substance and in particular for use in the treatment of thrombotic diseases.

The following Examples illustrate the invention.

Methods used for Examples

DNA cleavage

In general the cleavage of about 1µg of plasmid DNA or DNA fragments was effected using about 5 units of a restriction enzyme (or enzymes) in about 20µl of an appropriate buffer solution.

Generation of blunt ends:  If blunt ends were required they were produced by treating the DNA preparation with DNA Polymerase I, Klenow fragment (Molecular Cloning, A Laboratory Manual, Cold Spring Harbor Laboratory 1982).

Addition of 5' phosphate groups to oligonucleotides:

Addition of either labelled or unlabelled phosphate groups to oligonucleotides was carried out as described (Maxam and Gilbert, Methods in Enzymology Vol.65 p499-560  Ed. Grossman and Moldave publisher Academic Press 1980 or Molecular Cloning, A Laboratory Manual op.cit.).

Ligation of DNA Fragments:  Ligation reactions were carried out as described (Maniatis et al Molecular Cloning - A Laboratory Manual, Cold Spring Harbor Laboratory 1982).

Transformation of M13 DNA into bacterial cells was carried out using treatment with calcium chloride (Cohen et al 1973, P.N.A.S. 69, 2110).

Transformation of plasmid DNA into E. coli HB101 cells was either as described by Hanahan (DNA Cloning Vol I Chapter 6, D.M. Glover ed. IRL Press, 1985) in Protocol 3 except that incubation with RFI was for 5 minutes, or used competent HB101 cells supplied by Gibco BRL (Paisley, Scotland).

Isolation of t-PA cDNA clones

RNA was isolated from the TRBM6 cell line (Browne et al 1985, Thrombosis and Haemostasis; 54; 422-424) using hot phenol/SDS extraction (M.R.D. Scott, 1982, Ph.D. Thesis, University of London) and messenger RNA purified using oligo dT cellulose chromatography. A TRBM6 cDNA library was established by synthesizing double-stranded cDNA using this messenger RNA essentially as described by M.R.D. Scott (Ph.D. Thesis 1982, University of London). This cDNA preparation was digested with BglII, size fractionated on a Biogel A150 column and ligated into a pAT153 vector which had been modified by introduction of a BglII linker at the NruI site; the resultant DNA clone was propagated in E. coli K12 DH1 cells. A t-PA-specific oligonucleotide probe (Browne et al, 1985; Gene 33, 279-284)was used to identify a t-PA cDNA clone in the cDNA library, this clone is known as pTR108, and carries a 2kb BglII fragment encoding the mature t-PA protein (Pennica et al, 1983, Nature, 301, 214-221 or Molecular Cloning, A Laboratory Manual op. cit.).

A further cDNA clone λTR10, was isolated from a second TRBM6 cDNA library. The cDNA library was constructed using the λgt10 vector as described in 'DNA Cloning' Volume I, Chapters 2 and 3 (Ed. D.M. Glover (IRL Press, 1985)). The library was screened using appropriate t-PA specific oligonucleotide and plasmid probes using methods described previously (Browne et al, 1985, Gene, 33, 279-284). Clone λTR10 isolated from this library carries additional DNA 5' to that in pTR108, corresponding to the prepro-coding region and part of the 5' untranslated region (Pennica et al, 1983, Nature, 301, 214-221).


Growth of M13 single strand DNA: A single M13 phage plaque was picked into a 1:100 dilution in 2YT (1.6% Bactotryptone, 1% Yeast extract, 1% NaCl) of a fresh overnight culture of E. coli strain JM 101 (Gronenborn et al, 1976, Mol. Gen. Genet. 148, 243-250). The culture was grown at 37°C with shaking for 5-6 hours. The bacteria were pelleted and the supernatant retained. To this was added 200µl of 2.5M NaCl, 20% PEG6000 and the mixture was incubated at room temperature for 15 minutes. The mixture was centrifuged for 5 minutes in an Eppendorf microfuge and the resulting phage pellet was resuspended in 100µl of 10mM Tris pH 7.9, 0.1mM EDTA. After phenol extraction the phage DNA was precipitated with ethanol. The DNA pellet was washed with 70% ethanol, air dried and resuspended in 30µl of 10mM Tris pH 7.9, 0.1mM EDTA.

Growth of double stranded M13 DNA: A single M13 phage plaque was picked into 1ml of 2YT and grown with shaking at 37°C for 6 hours. The bacteria were pelleted and the supernatant containing M13 phage retained. Meanwhile a one litre 1:100 dilution of an overnight culture of E. coli strain JM 101 was grown at 37°C with shaking for 2 hours. 500µl of the M13 phage supernatant was added and the culture was shaken at 37°C for a further 4 hours. Preparation of double stranded DNA was carried out as described by Maniatis et al (Molecular Cloning, A Laboratory Manual, Cold Spring Harbor Laboratory, 1982).

Site directed mutagenesis:
The following double priming mixture was set up in a total volume of 5.6µl: 0.3pmoles template DNA (mTR 30 single stranded form, described in EP-A-0201153); 0.55 pmoles kinased mutagenic primer; 0.85 pmoles kinased M13 specific primer (Collaborative Research Inc., Product No. 20205); 0.6μl 10 x seq. buffer (0.1M Tris-Cl pH 7.5, 0.05M $MgCl_2$). The mixture was heated in an Eppendorf tube at 56°C for 6 minutes and then allowed to cool to room temperature for at least 15 minutes. To the priming mixture was added 6μl of the following mixture:- 1μl of 10mM solution of dATP, dGTP, dCTP, dTTP, (4μl total); 2μl 10mM rATP; 2μl 10 x seq. buffer; 12 μl $H_2O$; 2 units T4 DNA ligase; 2 units DNA Polymerase I (Klenow fragment). The mixure was incubated for 4 h at 25°C.

The DNA was transformed in 1μl aliquots into E. coli strain BMH 71-18 mut L (Kramer et al, 1984, Cell 38, 879-887). The transformed bacteria were plated onto a lawn formed from E. coli strain JM 101. Some of the plaques resulting from the transformations were picked and plated to form duplicate gridded arrays of bacterial colonies. Tnese were lifted onto nitro-cellulose and lysed as described (Grunstein and Hogness, 1975, P.N.A.S. 72, 3961).

Screening conditions: The nitrocellulose filters were prehybridised for 2h at 30°C in 6 x SSC (1 x SSC is 150mM NaCl, 15mM tri-sodium citrate 0.1% SDS, 10 x Denhardt's (Denhardt's is 0.02% Polyvinyl-pyrrolidone, 0.02% Ficoll, 0.02% BSA), 100μg/ml heat denatured, sonicated salmon sperm DNA. The filters were then mixed with a radio-actively labelled oligonucleotide (a shortened version of the mutagenic primer, 5′d(AAGTAGCACGTGGCCC) 3′ under the same buffer conditions and were hybridised overnight at 32°C.

- 28 -

The filters were washed at a series of different temperatures in 6 x SSC plus 0.1% SDS:  3 x 5 minutes at 42°C; 5 minutes at 52°C; 5 minutes at 56°C.  After each wash the filters were exposed, wet (with an intensifying screen) to Fuji RX-100 X-ray film.

Sequencing: DNA sequencing was carried out by the dideoxy termination method (Sanger, Nicklen and Coulson, 1977, P.N.A.S. 74, 5463).

Plasmid preparation:  Large scale preparation of plasmid DNA and plasmid mini-preparations were carried out as described in Maniatis et al., (Molecular Cloning - A Laboratory Manual, Cold Spring Harbor Laboratory, (1982).

Isolation of DNA fragments from low-melting-point (LMP) agarose gels

DNA fragments were isolated from LMP agarose gels as described by Maniatis et al., (Molecular Cloning, A Laboratory Manual, 1982, Cold Spring Harbor).

Ligation of Synthetic Linkers to DNA

Synthetic linkers encoding restriction enzyme sites, were kinased and ligated to blunt-ended DNA as described by Maniatis et al., (Molecular Cloning, A Laboratory Manual, 1982, Cold Spring Harbor).

Dephosphorylation of DNA

Vector DNA was dephosphorylated, where appropriate, by treatment with calf intestinal alkaline phosphatase as described by Maniatis et al (Molecular Cloning, A Laboratory Manual, 1982, Cold Spring Harbor).

<u>Expression of mutant t-PA</u>

1.

Cell preparation: Hela cells were trypsinised and plated out at a density of 1 to 2 x $10^4$ per $cm^2$ and left in growth medium (10% Serum (021-6010), 1% stock solution of penicillin/streptomycin (043-5070), 2% sodium bicarbonate (043-5080), 1% stock Glutamine (043-5030) in RPMI 1640 medium (041-2400); Gibco, Paisley, Scotland) at 37°C in a humidified incubator in an atmosphere of 5% $CO_2$/95% air. After 72h the cells were re-fed; they were used for DNA transfection after a further 24h.

Transfection procedure: The transfection (in Eagles MEM) used calcium coprecipitation as described in 'DNA Cloning' Ed. D.M. Glover (Chap. 15, C. Gorman). Glycerol shock and 5mM butyrate treatments were used. Plasminogen activator secreted by transfected cells was harvested in RPMI 1640 medium (as above but serum-free, containing 4% soybean peptone (Sigma)) for 24h and activity was measured using fibrin plates with reference to normal t-PA (J.H. Robinson, Thromb. Res., 1984; 33, 155).

2.

Cell preparation: mouse L929 cells were trypsinised and plated out at $10^6$ cells per 60mm dish and left overnight in growth medium (10% Serum, 1% stock solution of penicillin/streptomycin; Flow Laboratories, 1% Glutamine, 1% stock solution of non-essential amino acids, in Eagles MEM; Flow Laboratories) at 37°C in a humidified incubator in an atmosphere of 5% $CO_2$/95% air.

Transfection procedure: The transfection used (calcium coprecipitation) is described in 'DNA Cloning' Ed.

D.M. Glover (Chap. 15, C. Gorman). Glycerol shock and 10mM butyrate treatment were used.

Overlay: Agarose (Indubiose A$_{37}$), 2.4g in 95ml Eagles MEM (Gibco) heated to melting point in a bunsen flame, was then placed in a water bath maintained at 48$^{\circ}$C. 5.6 ml of fibrinogen (20mg/ml) were diluted 1:1 with 5.6 ml of Eagles MEM (containing an extra 7 mg/ml of NaCl) and retained on ice. 3.3 ml of Eagles MEM (no additions) were aliquoted into a bijou containing 86μl of bovine thrombin at 50 NIH Units/ml (retained on ice). The cells were washed 3 times with Eagles MEM to remove serum. The thrombin and fibrinogen were warmed to 37$^{\circ}$C in a water bath. 9.5 ml agarose were aliquoted into a pre-warmed universal. The thrombin was added to the agar, followed by the fibrinogen. The solution was poured over the cell layer, allowed to gel, and incubated at 37$^{\circ}$C until lysis zones appeared.

3.    Bowes Melanoma cells were seeded at a density of 2.6 x 10$^5$ cells per 35 mm dish and left overnight in growth medium (as for L929 cells). Transfection was as for L929 cells except that treatment with sodium butyrate was omitted. After glycerol shock the cells were left overnight in growth medium. The medium was replaced with serum-free harvest medium (MCDB-103 (041-0666), plus 1% stock solution of penicillin/streptomycin, 1% stock glutamine; Gibco, Paisley, Scotland).

Example 1

Construction of t-PA mutein cDNA lacking the growth
factor and first kringle domains

1. Preparation of DNA encoding the t-PA finger domain

6µg of the plasmid pTRE24 (described in EP-A-0207589)
was digested with 19.4 units of the enzyme MaeII
(Boehringer) for 2½h at 50°C in a volume of 75µl using
the buffer described by the manufacturer.

The MaeII cut DNA was flush-ended using DNA polymerase
I Klenow fragment. Kinased BamHI linkers (0.1 $A_{260}$
units; Amersham DC1203) of sequence 5'CCCGGATCCGGG3'
were ligated to the flush-ended MaeII fragments. After
ligation excess linkers were removed and cohesive
'sticky-ends' created by digestion with BamHI. The
BamHI digest was electrophoresed on a 1% low melting
point agarose gel. On this gel there were two bands
with mobility between that of the 1353 and 872 base
pair fragments found in a lambda DNA-HindIII/ØX-174RF-
HaeIII digest (Drigest TMIII, Pharmacia). The largest
of these bands, approximately 1100bp, was recovered (no
special attempt was made to ensure that other bands of
similar mobility did not contaminate this preparation, since undesired molecules are eliminated in the
cloning strategy). The 1100bp fragment was digested
with HindIII to create the desired approx. 360bp fragment with a HindIII 5' sticky end and a 3' BamHI sticky
end (converted from the MaeII site).

This finger domain-coding DNA fragment was sub-cloned
into plasmid pTRE12 (described in EP-A-0207589) between
the HindIII and BamHI sites, creating plasmid pTR6F.

- 32 -

The small HindIII-BamHI fragment (fragment I) in pTR6F encodes part of the 5' untranslated region of the t-PA cDNA, the signal/pre-pro region and residues 1 to 50 of the mature t-PA sequence followed by residues 88 to 91 of the mature t-PA sequence. Use of the BamHI linker (as described below) also introduces a further two amino-acids (proline-glycine).

2. Preparation of DNA fragment encoding kringle 2 region and part of B-chain

The t-PA cDNA has a unique SstI site at a position corresponding to nucleotide 1417 in the Pennica sequence (Nature 301, p214). There are several MaeIII sites but one located at nucleotide 722 is useful in constructing the t-PA mutein described below since there are no MaeIII sites between nucleotides 722 and 1417.

Plasmid pTRE15 (EP-A-0201153) was digested with the restriction enzymes MaeIII and SstI and a band of approx. 695bp was isolated from an agarose gel. No special care was taken to prevent contamination by fragment(s) with similar or identical mobility on this gel since these are eliminated in the cloning strategy. The MaeIII- SstI fragment is known as fragment II.

3. Preparation of oligonucleotide linker III

Two oligonucleotides of sequence:

                5' GATCCGAGGGAAACA 3'   (A)

                5' GTCACTGTTTCCCTCG 3'  (B)

were made on an Applied Biosystems DNA Synthesizer according to the Manufacturer's instructions. The oligonucleotides were combined and kinased prior to ligation.

4. Preparation of Vector DNA

Plasmid pTRE15 was digested with HindIII and SstI and the large approx. 6kb fragment (fragment IV) was isolated from an agarose gel. This fragment carries part of the t-PA B-chain-coding region and 3' untranslated region in addition to vector functions.

5. Construction of mutein DNA

The following DNA molecules were ligated together; (i) fragment I (isolated from pTR6F after HindIII plus BamHI digestion) (ii) linker III, (iii) fragment II and (iv) fragment IV. The ligated DNA was transformed into E. coli HB101.

Analysis of plasmids isolated from individual clones indicated that molecules of the desired structure (plasmid pTRE017) had been obtained.

Joining of fragment 1 to fragment II with linker III creates a DNA molecule encoding t-PA A-chain lacking the growth factor and kringle 1 domains, and part of the t-PA B-chain.

Linker III was designed to encode the 6 amino acid 'bridge sequence' connecting kringle 1 to kringle 2 in native t-PA; a single (silent) nucleotide change allowed the introduction of a BamHI site.

The junction of the finger-domain-coding region with the kringle 2 domain coding-region is shown in the following Table:

TABLE

Junction of finger domain-coding region with kringle 2 domain-coding region

```
        ←———————— fragment I ———————————→←———— linker III ————→←—— fragment II ——— - - -
                                        ←BamHI→
                                         linker
                                    **          +
5' .... AAA  AGT  ACC  AGG  GCC  ACG : CCC  GGA  TCC  GAG  GGA  AAC  AGT  GAC  TGC  TAC  .... 3'
3' .... TTT  TCA  TGG  TCC  CGG  TGC : GGG  CCT  AGG  CTC  CCT  TTG  TCA  CTG  ACG  ATG  .... 5'
        lys  ser  thr  arg  ala  thr   pro  gly  ser  glu  gly  asn  ser  asp  cys  tyr
        49   50   88   89   90   91                174  175  176  177  178  179  180  181
                                                    ↑                        ↑
                                                 BamHI site            reconstructed
                                                                       MaeIII site
```

\* = nucleotides introduced by flush-ending of MaeII site

† = nucleotide change (in triplet corresponding to amino acid 174 of mature t-PA) used to form BamHI site

Joining of fragment II to fragment IV (via reconstruction of the SstI site) allows reformation of the B-chain-coding region.


6.  Expression of mutein DNA

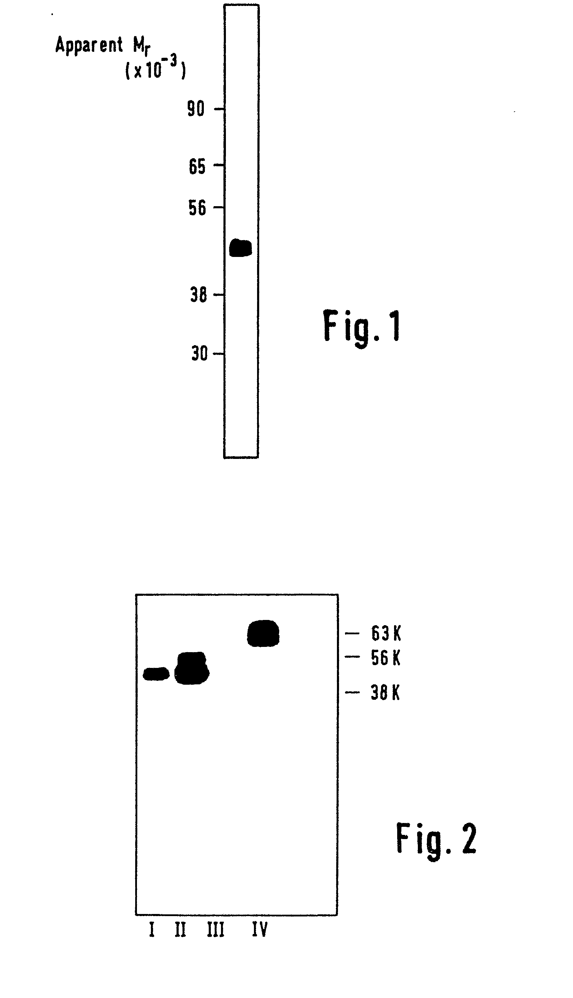

Plasmid pTRE017 was transfected into human HeLa cells as described in the Methods section and plasminogen activator activity was detected using the fibrin plate assay.  A sample of the harvest medium was analysed by fibrin zymography (Fig. 1) as described by Dodd et al., (1986) Thrombosis and Haemostasis 55(1); p94.  The activator had a mobility consistent with the predicted protein structure.

Example 2

Oligodeoxyribonucleotide site-directed mutagenesis to produce t-PA mutein cDNA lacking the first kringle domain.

1.   Preparation of oligonucleotide
5'd(TTCCCAAAGTAGCACGTGGCCCTGGT)3'

The above mentioned 26-mer was prepared by the solid phase phosphotriester method on a Bio Search Sam One Series II automated DNA synthesiser using monomer building blocks following the standard procedure described by the manufacturer.

The 26-mer was purified by ion-exchange HPLC using standard methodology as described in 'Chemical and Enzymatic Synthesis of Gene Fragments - A Laboratory Manual', Chapter 1, Verlag Chemie, Weinheim (1982).

The length of the oligonucleotide was verified by a
sizing experiment in which the electrophoretic mobility
of the oligonucleotide was compared with that of oligo
(dT) fragments contained in a commercially available
oligo (dT) ladder.

## 2.    Site-directed mutagenesis

The oligodeoxyribonucleotide prepared in 1. was used to
produce DNA coding for a novel modified t-PA protein.

The oligodeoxyribonucleotide is complementary to the
nucleotides 451-462 and 727-740 inclusive of the t-PA
cDNA (Pennica et al., Nature, 1983, 301, 214-221).  The
nucleotides complementary to 463-726 inclusive are not
present in this oligodeoxyribonucleotide and its use in
site-directed mutagenesis is intended to delete 88
codons from the t-PA coding sequence.

The site directed mutagenesis was carried out as
described above in Methods.

DNA sequencing was carried out using the dideoxy
termination method (Sanger et al., P.N.A.S., 1977, 74,
5463).  The nucleotides up to position 462 in the
mutant DNA have been shown to be adjacent to
nucleotides 727 onwards of the t-PA cDNA sequence.
This sequencing data has confirmed that the nucleotides
463-726 have been deleted from the t-PA gene.

## 3.    Expression of t-PA mutein in Eukaryotic Cells

The approx. 2kb BglII fragment encoding the mature
modified or unmodified (control) t-PA was excised from
the appropriate vector, and cloned into the unique BglII site of the expression vector pTRE12 (described in EP-A-0201153) using standard restriction endonuclease digestion and ligation techniques described in 'Molecular Cloning - A Laboratory Manual' by T. Maniatis, E.F. Fritsch and J. Sambrook, Cold Spring Harbor Laboratory (1982). Recombinant plasmids were characterised by restriction endonuclease digestion and mapping to identify isolates carrying the BglII fragment in the correct orientation for expression; these were called pTRE15 (unmodified t-PA) or pTRE49 (modified t-PA). Plasmid DNA prepared from recombinant E. coli by standard techniques was used to transfect cultured mouse L929 cells by the calcium phosphate precipitation technique essentially as described in 'High Efficiency Gene Transfer into Mammalian Cells' by C. Gorman (In 'DNA Cloning Volume II', ed. D. Glover, IRL Press Ltd., 1985).

Expression of modified and unmodified t-PA from transfected mouse L929 cells (24 hours post-transfection) was detected using the fibrin/agarose overlay technique essentially as described by Jones et al., Cell, 1975, 5, 323-329. Lytic zones were obtained from pTRE15 (unmodified t-PA) and pTRE49 (modified t-PA) transfected cells but not from cells transfected with the empty parent vector pTRE12 (t-PA$^-$).

The protein produced in the transient expression system was also examined by fibrin zymography. Following butyrate treatment the cell monolayers were washed twice with serum-free harvest medium and then incubated for 24h in serum-free medium. A sample of medium from each culture was then subjected to fibrin zymography as described in Dodd et al, (1986), Thrombosis Haemostasis 55 (1); 94. It is evident (Fig. 2) that deletion of kringle 1 results in a protein of smaller size than mature t-PA, it is also apparent that more than one species of activator is present in each medium sample presumably reflecting differences in post-translational modifications.

The modified t-PA was expressed in human cells. Expression in Hela cells was as described in Methods. Zymographic analysis of the expressed product revealed a major doublet of approximate Mr 53,000/55,000. Expression in the Bowes melanoma cell line (D.C. Rijken and D. Collen, 1981, J. Biol. Chem. 256, 7035) was as described in Methods. Zymographic analysis revealed the presence of modified t-PA with Mr similar to that of the Hela cell product (as well as the endogenous activator species).

Example 3

Oligodeoxyribonucleotide site-directed mutagenesis to produce t-PA mutein cDNA lacking the growth factor and first kringle domains.

1.    Preparation of oligonucleotide 5'd(TGTTTCCCTCAGAACTTTTGACAGGC)3'

The above mentioned 26-mer was prepared by the solid phase phosphoramidite method on an Applied Biosystems Model 381A automated DNA synthesiser using monomer building blocks following the standard procedure described by the manufacturer.

The fully deprotected oligonucleotide was used without subsequent purification.

- 39 -

2.  <u>Site-directed mutagenesis and expression of t-PA</u>
<u>mutein</u>

The oligonucleotide obtained in 1. was employed in a
deletion mutagenesis experiment as described in Nucl.
Acid. Res.; 1984, <u>12</u>, 2407 or Biochem. Soc. Trans.,
1984 <u>12</u>, 224.

Expression of the modified cDNA in a prokaryote or
eukaryote host will produce modified t-PA of the
invention.

<u>In the figures:</u>

Figure 1:  <u>Fibrin zymography of eukaryotic cell harvest</u>
           <u>medium</u>

           Medium from Hela cells transfected with
           plasmid pTRE017.

Figure 2:  <u>Fibrin zymography of eukaryotic cell harvest</u>
           <u>media.</u>

Media samples from L929 cells transfected with plasmids
pTRE15, pTRE49 or pTRE12.

Fibrinolytic activity was detected in lanes I and II
(two different concentrations of pTRE49 modified t-PA)
and lane IV (pTRE15, unmodified t-PA).  Harvest medium
from cells transfected with the empty pTRE12 vector
(t-PA$^-$) was run in lane III (63K, 56K and 38K indicate
the approximate position of size markers).

C

CLAIMS

1.  A fibrinolytically active tissue-type plasminogen activator which has been modified in the region of the first kringle domain.

2.  A modified tissue-type plasminogen activator according to claim 1, which has been modified in the region from amino acid residues 85 to 179.

3.  A modified tissue-type plasminogen activator according to claim 1 or 2, wherein the modification comprises the deletion of all or part of the first kringle domain.

4.  A modified tissue-type plasminogen activator according to claim 3, wherein the modification comprises the deletion of amino acid residues 92 to 179 inclusive.

5.  A modified tissue-type plasminogen activator according to claim 1, which has also been modified in the region of the growth factor domain.

6.  A modified tissue-type plasminogen activator according to claim 5, which has been modified in the region from amino acid residues 51 to 179 inclusive.

7.  A modified tissue-type plasminogen activator according to claim 5 or 6, wherein the modification comprises the deletion of all or part of the first kringle and growth factor domains.

- 2 -

8.    A modified tissue-type plasminogen activator according to claim 7, wherein the modification comprises the deletion of amino acid residues 51 to 87 and 92 to 173 inclusive.

9.    A modified tissue-type plasminogen activator according to claim 8, wherein the residues 92 to 173 are replaced by -proline-glycine-.

10.    A modified tissue-type plasminogen activator according to claim 7, wherein the modification comprises the deletion of amino acid residues 51 to 173 inclusive.

11.    A modified tissue-type plasminogen activator prepared according to Example 1, 2 or 3.

12.    A derivative of the modified tissue-type plasminogen activator according to any of claims 1 to 11.

13.    A DNA polymer comprising a nucleotide sequence that encodes a modified tissue-type plasminogen activator according to any of claims 1 to 11.

14.    A replicable expression vector capable, in a host cell, of expressing a DNA polymer according to claim 13.

15.    A host cell transformed with the vector according to claim 14.

16.    An oligodeoxyribonucleotide comprising a nucleotide sequence which is complementary or corresponds to a region of the t-PA cDNA extending 5′

from nucleotide 462 inclusive, said sequence being
joined directly to a nucleotide sequence which is
complementary or corresponds to a region extending 3'
from nucleotide 727 inclusive of t-PA cDNA.

17. The oligodeoxyribonucleotide having the sequence
(V):

5'd(TTCCCAAAGTAGCACGTGGCCCTGGT)3'          (V)

18. An oligodeoxyribonucleotide comprising a
nucleotide sequence which is complementary or
corresponds to a region of the t-PA cDNA extending 5'
from nucleotide 339 inclusive, said sequence being
joined directly to a nucleotide sequence which is
complementary or corresponds to a region extending 3'
from nucleotide 709 inclusive of t-PA cDNA.

19. The oligodeoxyribonucleotide having the sequence
(VI):

5'd(TGTTTCCCTCAGAACTTTTGACAGGC)3'          (VI)

20. An oligonucleotide linker of structure (III):

5' GA TCC GAG GGA AAC A          3'
3'       G CTC CCT TTG TCA CTG 5'

21. t-PA A-chain which has been modified in accordance
with any of claims 1 to 10.

22. A DNA polymer comprising a nucleotide sequence
that encodes the modified t-PA A-chain according to
claim 21.

- 4 -

23. A hybrid protein comprising the modified t-PA A-chain according to claim 21 linked to the B-chain of a fibrinolytically active protease, the catalytic site of which is optionally blocked by a removable blocking group.

24. A pharmaceutical composition comprising modified tissue-type plasminogen activator according to any of claims 1 to 12 in combination with a pharmaceutically acceptable carrier.

25. A modified tissue-type plasminogen activator according to any of claims 1 to 12 for use as an active therapeutic substance.

26. A modified tissue-type plasminogen activator according to any of claims 1 to 12 for use in the treatment of thrombotic disease.

27. Use of a modified tissue-type plasminogen activator according to any of claims 1 to 12 for the preparation of a medicament for the treatment of thrombotic disease.

28. A process for preparing modified tissue-type plasminogen activator according to claim 1, which process comprises expressing DNA encoding said modified tissue-type plasminogen activator in a recombinant host cell and recovering the modified tissue-type plasminogen activator product.

29. Modified tissue-type plasminogen activator obtainable by the process of claim 28.

- 1 -

B

CLAIMS FOR SPAIN

1. A process for preparing a fibrinolytically active tissue-type plasminogen activator which has been modified in the region of the first kringle domain, which process comprises expressing DNA encoding said modified tissue-type plasminogen activator in a recombinant host cell and recovering the modified tissue-type plasminogen activator product.

2. A process according to claim 1, in which the tissue-type plasminogen activator has been modified in the region from amino acid residues 85 to 179.

3. A process according to claim 1 or 2, wherein the modification comprises the deletion of all or part of the first kringle domain.

4. A process according to claim 3, wherein the modification comprises the deletion of amino acid residues 92 to 179 inclusive.

5. A process according to claim 1, in which the tissue-type plasminogen activator has also been modified in the region of the growth factor domain.

6. A process according to claim 5, in which the tissue-type plasminogen activator has been modified in the region from amino acid residues 51 to 179 inclusive.

7. A process according to claim 5 or 6, wherein the modification comprises the deletion of all or part of the first kringle and growth factor domains.

8. A proces according to claim 7, wherein the modification comprises the deletion of amino acid residues 51 to 87 and 92 to 173 inclusive.

9. A process according to claim 8, wherein the residues 92 to 173 are replaced by -proline-glycine-.

10. A process according to claim 7, wherein the modification comprises the deletion of amino acid residues 51 to 173 inclusive.

11. A process for preparing a DNA polymer comprising a nucleotide sequence that encodes a modified tissue-type plasminogen activator according to any of claims 1 to 10, by the condensation of appropriate mono-, di- or digomeric nucleotide units.

12. A process for preparing a replicable expression vector capable, in a host cell, of expressing a DNA polymer as defined in claim 11, which process comprises cleaving a vector compatible with said host cell to provide a linear DNA segment having an intact replicon, and combining said linear segment with one or more DNA molecules which, together with said linear segment, encode the modified t-PA, under ligating conditions.

13. A process for preparing a host cell transformed with the vector as defined in claim 12, which process comprises transforming a host cell with a said vector under transforming conditions.

14. A process for preparing an oligodeoxyribonucleotide comprising a nucleotide sequence which is complementary or corresponds to a region of the t-PA cDNA extending 5'

- 3 -

from nucleotide 462 inclusive, said sequence being joined directly to a nucleotide sequence which is complementary or corresponds to a region extending 3′ from nucleotide 727 inclusive of t-PA cDNA, an oligodeoxyribonucleotide comprising a nucleotide sequence which is complementary or corresponds to a region of the t-PA cDNA extending 5′ from nucleotide 339 inclusive, said sequence being joined directly to a nucleotide sequence which is complementary or corresponds to a region extending 3′ from nucleotide 709 inclusive of t-PA cDNA, or an oligonucleotide linker of structure (III):

```
5' GA TCC GAG GGA AAC A        3'
3'        G CTC CCT TTG TCA CTG 5'
```

which process comprises the deprotection of a second oligodeoxyribonucleotide having the same sequence of bases as said oligodeoxyribonucleotide and in which all free hydroxy and amino groups are protected.

15.  A process according to claim 11, which process comprises effecting a site-directed mutagenesis upon the cDNA which codes for tissue-type plasminogen activator.

16.  A process according to claim 15, wherein the site-directed mutagenesis is effected using an oligodeoxyribonucleotide as defined in claim 14.

17.  A process according to claim 11, which process comprises ligating a first DNA molecule comprising a DNA sequence encoding the finger domain amino acid sequence with a second DNA molecule comprising a DNA sequence encoding the second kringle domain.

- 4 -

18. A process for preparing t-PA A-chain which has been modified in accordance with any of claims 1 to 10,which process comprises separating the A-chain of t-PA which has been so modified from the B-chain thereof by mild reduction.

19. A process for preparing t-PA A-chain as defined in claim 18, which process comprises expressing DNA encoding said modified A-chain in a recombinant host cell and recovering the modified t-PA A-chain product.

20. A process for preparing a hybrid protein comprising the modified t-PA A-chain as defined in claim 18 linked to the B-chain of a fibrinolytically active protease, the catalytic site of which is optionally blocked by a removable blocking group, which process comprises mixing said A- and B- chains under oxidative conditions and thereafter optionally blocking the catalytic site with a removable blocking group.

21. A process for preparing a protein as defined in claim 20, which process comprises ligating DNA encoding said A-chain to DNA encoding said B-chain, expressing the ligated DNA in a prokaryote or eukaryote host and thereafter optionally blocking the catalytic site of the expressed product with a removable blocking group.

22. Use of a modified tissue-type plasminogen activator according to any of claims 1 to 10 for the preparation of a medicament for the treatment of thrombotic disease.

23. A fibrinolytically active tissue-type plasminogen activator as defined in claim 1, whenever prepared by the process of claim 1 or an obvious equivalent thereof.

Apparent M$_r$
($\times 10^{-3}$)

90

65

56

38

30

Fig. 1

— 63 K
— 56 K
— 38 K

I   II   III   IV

Fig. 2

## EUROPEAN SEARCH REPORT

| | DOCUMENTS CONSIDERED TO BE RELEVANT | | EP 87302835.1 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,P, X | <u>EP - A1 - 0 207 589</u> (BEECHAM GROUP PLC) <br> * Claims 1-10,12-15,18-21 * | 1,2,5-8,10-19,21-29 | C 12 N 15/00 <br><br> C 12 N 9/68 <br><br> C 07 H 21/04 <br><br> C 12 N 5/00 <br><br> A 61 K 37/54 |
| P,X | <u>EP - A2 - 0 199 574</u> (GENENTECH, INC.) <br> * Claims 1,20 * | 1,25-27 | |
| A,D, P | <u>EP - A2 - 0 201 153</u> (BEECHAM GROUP PLC) <br> * Claims 1,11-14 * | 1,24-28 | |
| A | <u>WO - A1 - 86/01 538</u> (BIOGEN N.V.) <br> * Abstract * | 1 | |
| D,A | NATURE, vol. 301, January 20, 1983 (New York, London) <br><br> D. PENNICA et al. "Cloning and expression of human tissue-type plasminogen activator cDNA in E. coli" <br> pages 214-221 <br> * Totality * | 1,13-15,28,29 | TECHNICAL FIELDS SEARCHED (Int Cl 4) <br><br> C 12 N <br> C 07 H <br> A 61 K |
| D,A | BIOTECHNOLOGY, vol. 2, no. 12, December 1984 (New York) <br><br> G.A. VEHAR et al. "Characterization Studies on Human Melanoma Cell Tissue Plasminogen Activator" <br> pages 1051-1057 <br> * Totality * | 1 | |

The present search report has been drawn up for all claims

| Place of search | Date of completion of the search | Examiner |
|---|---|---|
| VIENNA | 24-06-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

## DOCUMENTS CONSIDERED TO BE RELEVANT

EP 87302835.1

| Category | Citation of document with indication, where appropriate, of relevant passages | Relevant to claim | CLASSIFICATION OF THE APPLICATION (Int Cl 4) |
|---|---|---|---|
| D,A | EP - A1 - 0 093 619 (GENENTECH, INC.)<br><br>* Claims 1-7,13-15; fig. 5 *<br><br>---- | 1,13-15,24-29 | |
| | | | **TECHNICAL FIELDS SEARCHED (Int Cl 4)** |

The present search report has been drawn up for all claims

| Place of search | Date of completion of the search | Examiner |
|---|---|---|
| VIENNA | 24-06-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82